# EUROPEAN PATENT SPECIFICATION

(11) **EP 0 499 417 B1**
(45) Date of publication and mention of the grant of the patent: **29.11.1995**
(21) Application number: 92301052.4
(22) Date of filing: 07.02.1992
(51) Int. Cl.: A61M 5/168, F04B 49/08

(54) **Pumping arrangements**
Pumpenanordnung
Agencement de pompage

(30) Priority: 14.02.1991 GB 9103165
(43) Date of publication of application: 19.08.1992
(73) Proprietor: BAXTER INTERNATIONAL INC., Deerfield, IL 60015-4633 (US)
(72) Inventor: Danby, Hal, Sudbury, Suffolk CO10 0PZ (GB)
(74) Representative: MacGregor, Gordon

(56) References cited:
- EP-A- 0 361 793
- WO-A-87/05225
- US-A- 4 277 226

## Description

This invention relates to pumping apparatus and in particular to pumping apparatus for use in medical applications such as the intravenous supply of fluids to a patient.

In such applications as last-mentioned it is common for deformable tubing such as p.v.c. tubing, to be used to deliver the required fluid to the point of application. Where the supply rate and pressure is required to be carefully controlled, as is generally the case in medical applications as aforesaid, a serious problem can arise if the delivery tubing is restricted in any way (for example, by a patient rolling over onto it). Means can be provided to detect the resultant increase in pressure in the delivery tubing and disable the pumping action. However, if the restriction is removed (in the example given by the patient rolling over again, off the tubing, or more likely by a nurse responding to an alarm also initiated by the means for detecting the increase in pressure), an undesirable, surge of fluid to the point of application can result due to pressure retained in the delivery tubing.

EP-A-0361793 discloses a pumping apparatus which can detect the increase in fluid pressure in tubing that arises as a result of flow restriction, and use such detection in order to activate an alarm which alerts the operator to the presence of fluid flow restriction in the tubing. EP-A-0361793 does not disclose any means in the apparatus which is operable to prevent an undesirable fluid surge in the tubing when the operator removes the cause of the fluid flow restriction.

The precharacterising part of Claim 1 is based on EP-A-0361793 and the distinguishing features of the present invention are set out in the characterising part of Claim 1.

According to the present invention there is provided a pumping apparatus for receiving tubing, the apparatus comprising pressurizing means for subjecting fluid in the tubing to pressure, first and second controllable valve means respectively on the input side and output side of the pressurizing means for restricting fluid flow, detecting means for detecting fluid pressure in the tubing and positioned on the output side of said second valve means, and control means responsive to said detecting means and operable to control both said valve means, characterised in that the control means comprises means operable to cause both of the valve means to open when increases in fluid pressure exceeding a given threshold are detected in the tubing, whereby fluid under excess pressure in said tubing is released back towards the first valve means.

Preferably means are also provided for disabling said fluid pressure subjecting means in response to increases in pressure detected by said fluid pressure responsive means.

Preferably said controllable valve means each comprises means operable to squeeze said tubing to produce occlusion.

The invention is illustrated in and further described with reference to the accompanying drawings, wherein:
Figure 1 is a schematic block diagram of a pumping arrangement in accordance with the present invention;
Figure 2 is a view in elevation of a fluid pressure responsive device;
Figure 3 shows a cross-section along the line x-x in Figure 2;
Figure 4 is a schematic block diagram of a liquid flow monitoring arrangement with a pressure responsive device;
Figure 5 illustrates in highly shematic fashion the cycle of operation of the controllable valves and pressurizing means; and
Figures 6 and 7 illustrate the nature and operation of the pressurizing means.

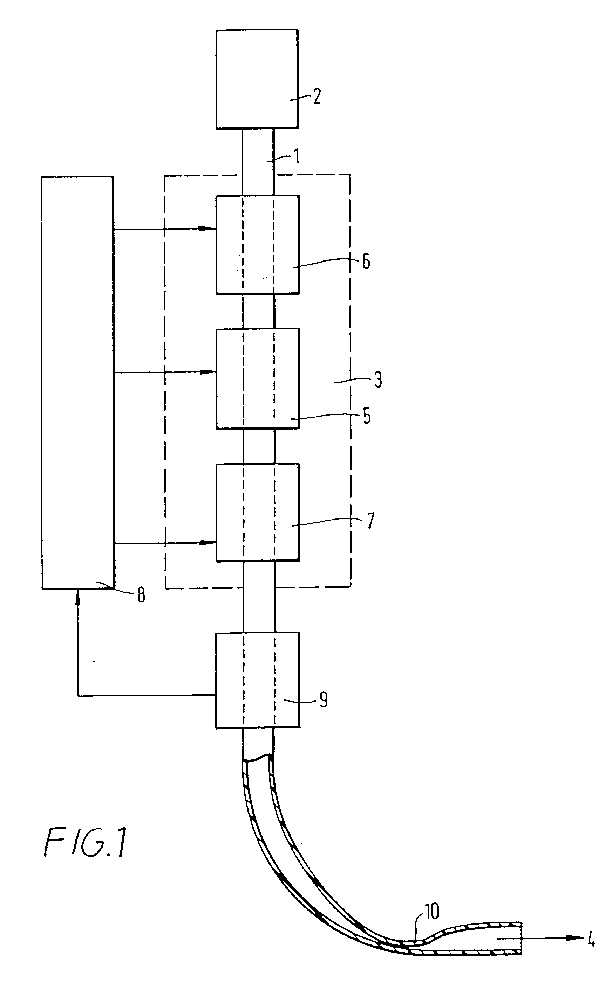

Referring to Figure 1, a length of standard pliant p.v.c. tubing 1 of approximately 4.1mm outside diameter and 0.5mm wall thickness is connected at one end to a source of fluid 2. From that end, tubing 1 passes through a pumping device 3 to a point of application 4. The point of application in this case is a patient and a fluid is a fluid required to be supplied intravenously. The means at the point of application for achieving this is not represented, but may be taken to be conventional.

Between the source of fluid 2 and pumping device 3 the tubing 1 may be regarded as supply tubing, whilst between pumping device 3 and the point of application 4 the tubing 1 may be regarded as delivery tubing, although in this example the length of tubing is continuous.

Pumping device 3 comprises a pressurizing means 5 (referred to hereafter as squeezing device 5) through which tubing 1 passes. Squeezing device 5 is arranged to deform tubing 1, first in one direction locally to reduce its volume and thereafter in another direction tending to restore the original cross-sectional shape of the tubing 1.

On the supply or input side of squeezing device 5 is a controllable input valve 6 which is operable to block the passage of fluid between the squeezing device 5 and the fluid source 2 by squeezing tubing 1 to produce occlusion.

On the delivery or output side of squeezing device 5 is a controllable output valve 7 which is operable to block the passage of fluid between the squeezing device 5 and the point of application 4.

Controllable valves 6 and 7 described herein correspond to inlet and outlet valves described in detail in EP-A-0426273.

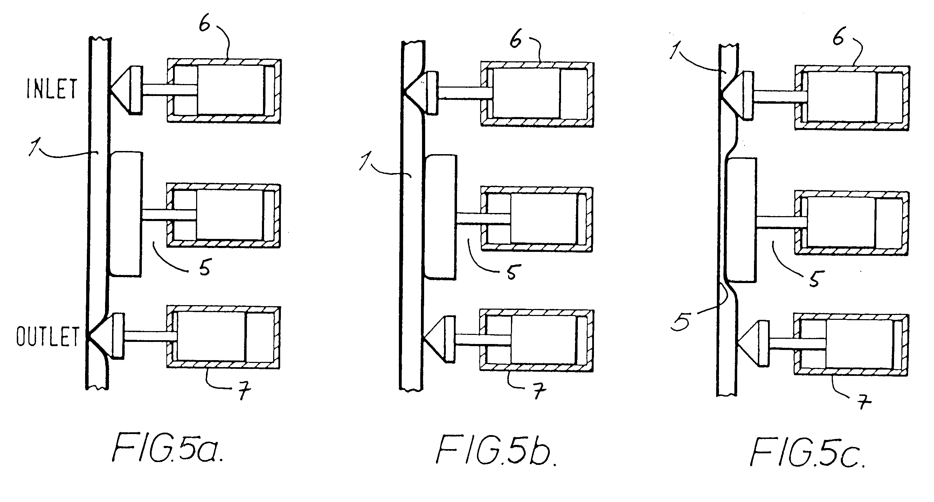

Squeezing device 5 and inlet and outlet valves 6 and 7 shown in the accompanying drawing are controlled by a microprocessor control unit 8 to produce the pumping action as follows. Briefly, as shown in Figure 5, with squeezing device 5 relaxed, output valve 7 closed and inlet valve open, fluid passes into the region of the tubing encompassed by squeezing means 5. With squeezing means 5 activated locally to reduce the volume of tubing 1, inlet valve 6 closed and outlet valve 7 open, fluid is passed towards the point of application 4. The process then repeats. Normally, it will be noted, inlet valve 6 is shut when outlet valve 7 is open and vice versa.

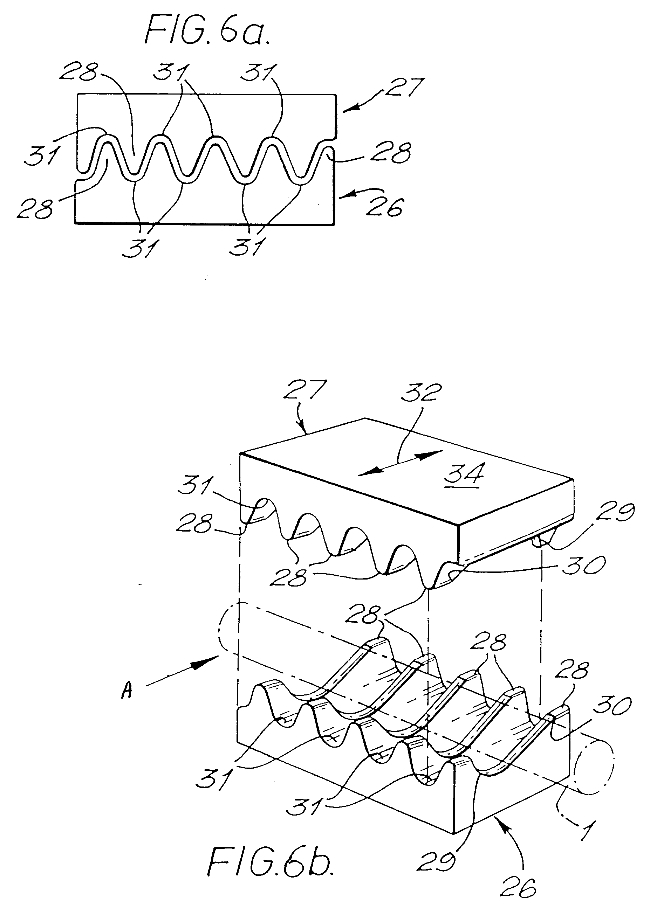

Referring to Figure 6 this illustrates at (b), by way of a perspective sketch, the two principal components of the squeezing means 5. The view shown in (a) is in the direction of the arrow "A" in (b) and shows the two principal-component members 26 and 27 of the deforming means united.

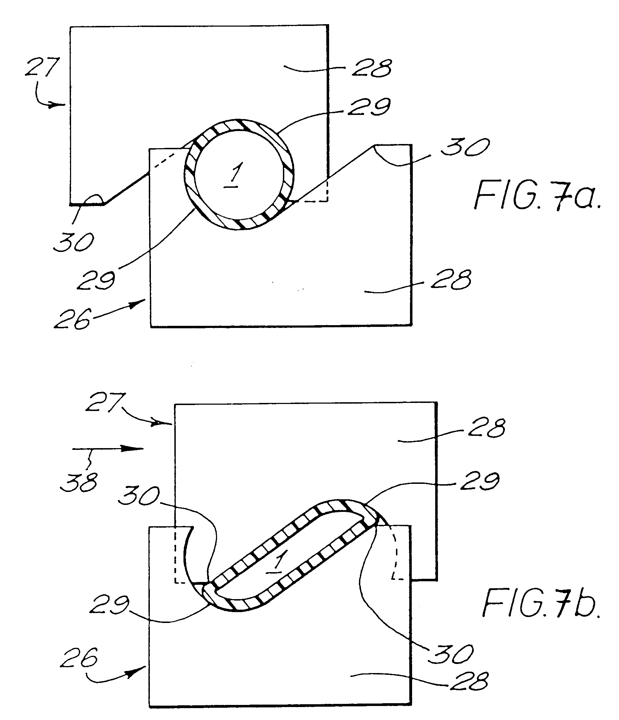

Member 26 has a series of transverse ridges 28 which are shaped to provide a valley through which the aforementioned tubing 1, again show in dotted outlet, may pass. As best illustrated in Figure 7, which demonstrates the action of the squeezing means 5, each ridge 28 has a recess which is semi-circular to one side 29 and of progressively decreasing depth towards the other side 30 of the recess until the full height of the ridge is reached. Viewed in the direction of the passage of the tubing 1 through the valley formed by the recessed in the ridges 28 all of the ridges of the member 26 appear superimposed one upon the other, with all of the semi-circular portions of the recesses to the same side.

The member 27 is generally similar to the member 26 (as reflected by the use of like reference numbers for like parts) except that it is relatively inverted with, as best seen from Figure 6 (a), the ridges 28 of one interdigitated with the ridges 28 of the other. Whilst in Figure 6 (a), for ease of illustration, a gap is shown between the two members 26 and 27, in practice the ridges 28 of each member 26 or 27 bear on the surfaces 31 between the ridges 28 of the other member 27 or 26. The member 26 is arranged to be stationary during operation whilst member 27 is arranged to move in the manner of a shuttle, to and fro as represented by the double headed arrow 32 in Figure 6. The effect of one cycle of movement of the member 27 relative to the member 26 is best seen from Figure 7. In (a) of Figure 7 the position of member 27 relative to member 26 is such that the semi-circular portions of the recesses in the ridges 28 of the two members 26,27 form a passage which is of substantially circular cross-section through the squeezing device in which the tubing 1 passes with no or no significant distortion. As member 27 moves in the direction of the arrow 38 (Figure 7 (b)), the tubing 1 is squeezed to produce a cross-section which is oval in shape and of reduced area. As the limit of movement of the member 27 in the direction of the arrow 38 is reached, the member 27 is moved in a reverse direction to the start position shown in Figure 7 (a). This cycle repeats continuously whilst the pumping device 3 is energised. The microprocessor control unit 8 controls the reciprocal movement of the members 26,27.

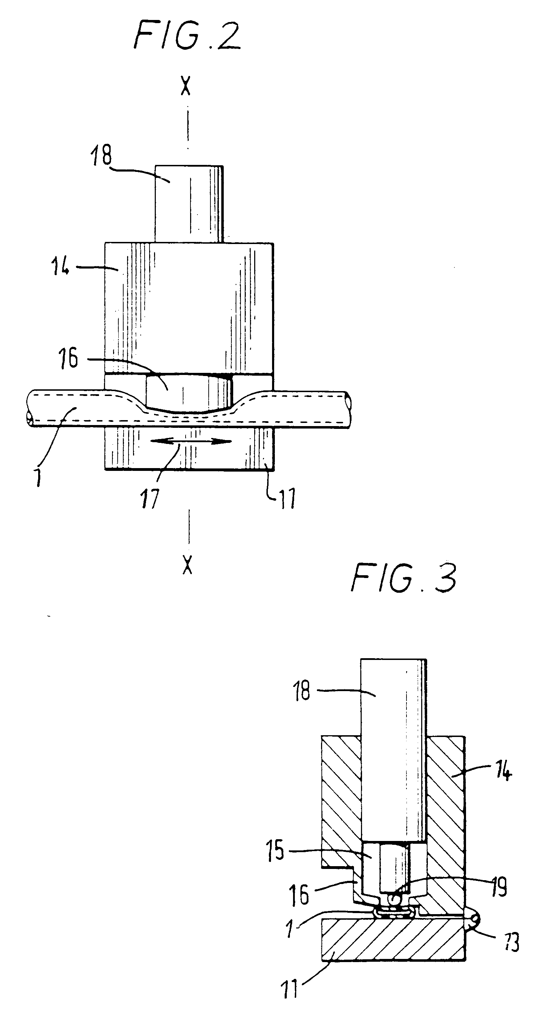

Beyond outlet valve 7 is a pressure responsive device 9 which is illustrated in Figures 2 and 3.

Referring to Figures 2 and 3, the device 9 illustrated comprises a hard bed member 11 across the surface of which may be positioned a length of pliant tubing 1.

Hinged to the bed member 11 by hinge 13 is a main body member 14 which has a circular cylindrical passage 15 extending orthogonally away from the surface of the bed member 11 across which said tubing 1 is positioned. The passage 15 exits from main body member 14 towards tubing 1 through a tubular projection 16 which, when the main body member 14 is closed up towards the bed member 11, flattens the tubing 1 against the surface of the bed member 11 over a short length 17 thereof. The extent to which tubular projection 16 projects towards bed member 11 is such that when the main body member 14 is fully closed up to the bed member 11 (and held by means of a suitable catch, not represented) the tubing 1 is flattened against the bed member 11 without occlusion occurring and without unduly impeding the flow of liquid through the tubing 1.

Inserted within the passage 15 is a linear voltage displacement transducer 18 of known form. As known per se transducer 18 has a spring-loaded plunger 19 and produces an output digital voltage signal which is indicative of the extent to which plunger 19 extends beyond the body of the transducer 18. The output voltage signal of transducer 18 will thus vary with the displacement of a surface against which plunger 19 is pressed in the direction of action of said plunger.

Transducer 18 is entered into and fixed within the passage 15 to an extent such that plunger 19 is spring-biased against the flattened surface of the tubing 1 when the main body member 14 is closed up and secured to the bed member 11, as previously described.

In operation, any variation in the pressure of fluid passing through the tubing 1 will tend to cause expansions and contractions. It is believed that flattening the tubing as above described magnifies the resultant displacement of the relatively flat portion of the wall of the tubing with which the plunger 19 is in contact, thus enabling the transducer 18 to respond to such variations with a higher degree of sensitivity than would otherwise be the case.

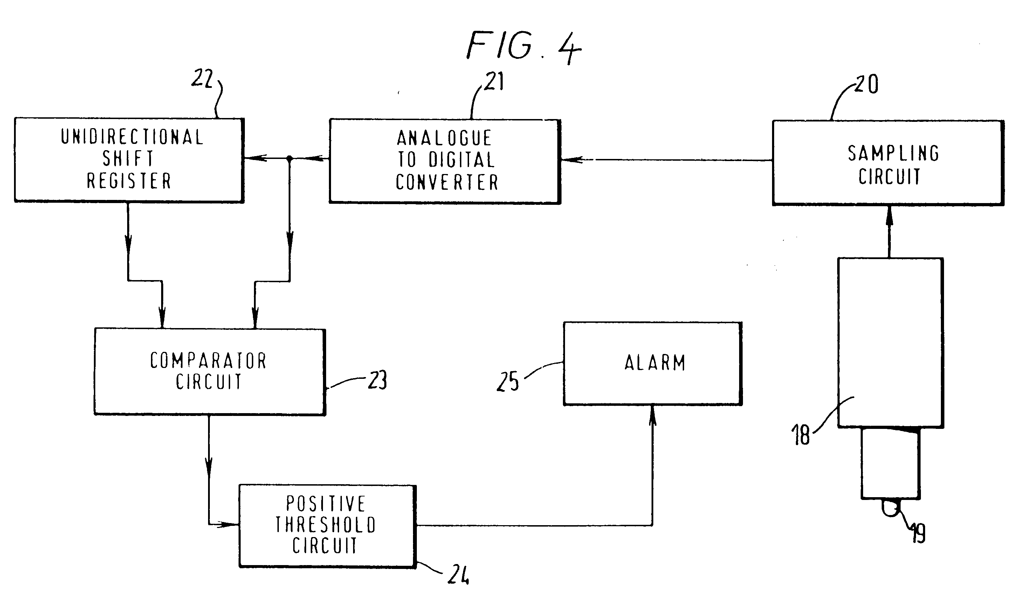

Referring to Figure 4, only transducer 18 and plunger 19 of the device of Figures 2 and 3 are represented. The analogue output voltage of transducer 18 is arranged to be sampled at the rate of once per second by a sampling circuit 20.

The output of sampling circuit 20 is connected to the input of an analogue-to-digital converter 21.

The output of analogue-to-digital converter 21 is connected to a unidirectional "count down" only shift register 22, where it is stored, and in parallel to one input of a comparator circuit 23. A second input for comparator circuit 23 is derived from the output of shift register 22 so that comparator circuit 23 compares the digital signal output of analogue-to-digital converter 21 derived from one sample taken by sampling circuit 20 with that derived from the sample taken one second before.

The output of comparator circuit 23 is connected via a positive threshold circuit to a suitable visual and/or aural alarm 25.

For further understanding of the operation of the arrangement illustrated in Figure 4, reference is made to EP-A-0471492.

As plunger 19 is pressed into the flattened portion 17 of the wall of the tubing 1, the wall will first yield markedly over a very short period of time and then, with main body 14 of the monitoring device fully closed up to bed member 11, will continue to yield further, but decreasingly so until a substantially stable state is reached.

Sampling is carried out at regular intervals from the time that pressure is first applied to said area and continuing beyond the point in time at which the aforementioned substantially stable state is reached.

In other embodiments the functions of components 20 to 24 are provided by a suitably programmed microprocessor.

In the embodiment illustrated in Figure 1, pressure responsive device 9 is connected to microprocessor control unit 8. When the signal from pressure responsive device 9 exceeds a given threshold indicating that the pressure in the delivery portion of tubing 1 had risen beyond a predetermined value indicative of a restriction 10 caused, for example, by the patient rolling over onto the tubing 1, control unit 8 acts to cause squeezing device 5 to be disabled and both input and output valves to be opened together so as to enable excess fluid in the delivery portion of tubing 1 to pass back towards the fluid source 2 and thus relieve the excess pressure caused by the restriction. At the same time an alarm is caused to be activated to call the attention of a nurse to the restriction.

It will be appreciated that the arrangement may be such that normal operation of the pumping device 3 recommences as pressure in the delivery portion of tubing 1 reduces. In that case, however, "hunting" may occur since a reduction in pressure will occur not only because of a removal of the causative restriction, but also by the very opening of valves 6 and 7 upon device 9 detecting an increase in pressure.

If "hunting" as described is undesirable, renewed pumping may be subject to a reset control, operable by the nurse upon removing the cause of the restriction.

## Claims

1. A pumping apparatus for receiving tubing (1), the apparatus comprising pressurizing means (5) for subjecting fluid in the tubing (1) to pressure, first and second controllable valve means (6,7) respectively on the input side and output side of the pressurizing means (5) for restricting fluid flow, detecting means (9) for detecting fluid pressure in the tubing (1) and positioned on the output side of said second valve means (7), and control means (8) responsive to said detecting means (9) and operable to control both said valve means (6,7), characterised in that the control means (8) comprises means operable to cause both of the valve means (6,7) to open when increases in fluid pressure exceeding a given threshold are detected in the tubing (1), whereby fluid under excess pressure in said tubing (1) is released back towards the first valve means (6).

2. The apparatus of Claim 1, wherein the control means (8) is operable to disable said pressurizing means (5) in response to increases in fluid pressure in the tubing detected by said detecting means (9).

3. The apparatus of Claim 1 or 2, wherein each valve means (6,7) is operable to squeeze said tubing (1) to produce occlusion thereof.

4. Apparatus as in any one of Claims 1 to 3 in combination with tubing (1) connected between a fluid source (2) and a point (4) of application of the fluid.

5. Pumping apparatus as in any preceding claim, wherein the pressurizing means (5) includes first and second members (26,27) defining a chamber receiving the tubing (1), the members (26,27) being relatively reciprocable to cause alternate deformation and reformation of the tubing (1) in a pumping action wherein the volume of the tubing (1) is reduced when it is deformed, the apparatus including means for urging the tubing (1) to its reformed state in which the normal cross-sectional shape of the tubing (1) is restored, wherein in a first relative position of the members (26,27), the chamber is substantially cylindrical, whereby the members (26,27) urge the tubing (1) to its reformed state, and the members (26,27) being relatively movable in a direction radial of the chamber and tubing (1) to a second relative position, in which the chamber is elongate to cause deformation of the tubing (1).

6. A pumping apparatus as in Claim 5 wherein said control means (8) is operable to control the reciprocal movement of said members (26,27) and the operation of said valve means such that when said tubing is being deformed by said members (26,27), the valve means (6) on the upstream side restricts fluid flow through said tubing (1), whilst the valve means (7) on the downstream side permits increased liquid flow and when said tubing is being urged to its reformed state, the valve means (7) on the downstream side restricts fluid flow through the tubing (1) whilst the valve means (6) on the upstream side permits increased liquid flow.

7. A pumping apparatus according to any preceding claim, wherein the detecting means (9) comprises first means (11,16) for causing the tubing (1) to assume an elongate cross-section over a length thereof to define a relatively flattened region of a wall of the tubing (1) within said length, second means (18,19) for applying pressure to a part of the relatively flattened region, the second means being responsive to movement of said part to provide indications of variations in the pressure of said fluid, and means (20-25) for compensating for movement of said part occurring after said pressure is applied thereto and due to other than variations in the pressure of said fluid.

8. A pumping apparatus as in Claim 7 wherein said compensating means (20-25) comprises means (23) for comparing an indication derived at a given point in time with an indication derived at a previous point in time and means (24) responsive to the existence therebetween of a difference above a threshold level, for signalling that a change in the pressure of said fluid has occurred.

9. An apparatus as claimed in Claim 7 or 8 and wherein said first means (11,16) for causing said tubing (1) to assume an elongate cross-section over a length thereof comprises a bed member (11) having a non-resilient substantially flat surface across which said tubing (1) may be positioned and a body member (14,16) for flattening said tubing (1) non-occlusively over a length thereof against said surface of said bed member and the second means (18,19) comprises a pressure applying member (18) carried by the body member (14,16) for applying pressure to a point in said relatively flattened region.

10. An apparatus as claimed in Claim 9 and wherein said pressure-applying member (18) comprises a linear voltage displacement transducer having a spring-loaded plunger (19) and producing, in operation, an output voltage signal which is indicative of the extent to which said plunger extends beyond the body of said transducer, said plunger (19) being arranged to be pressed into the relatively flattened region of the wall of said tubing at said point.

11. An apparatus as claimed in Claim 10 and wherein said transducer (18) is housed in the body member (14,16) which is arranged to be movable towards and away from said bed member (11) whereby said tubing (1) may be engaged or released by said plunger (19).

12. An apparatus as claimed in Claim 11 and wherein said body member (14,16) includes a tubular projection extending beyond a cylindrical passage (15) within said body member, said passage opening in a surface of said body member facing said bed member (11) and said tubular projection (16) serving to flatten said tubing as aforesaid as said body member (14,16) is moved towards said bed member (11).

13. An apparatus as claimed in Claim 11 or 12 and wherein said body member (14,16) and said bed member (11) are hinged together and means are provided for securing the one to the other when the body member is closed up to said bed member in operation.

14. An apparatus as claimed in any of Claims 10 to 13 and wherein the output of said linear voltage displacement transducer (18) is arranged to be sampled by a sampling circuit (20), the output of which is connected via an analogue-to-digital converter (21), to the input of a unidirectional shift register (22) and in parallel to one input of a comparison circuit (23), a second input of which is derived from the output of said shift register (22), whereby the digitalised output of said sampling circuit is compared with the immediately preceding output of said sampling circuit outputted from said shift register, the output of said comparison circuit (23) being connected via a threshold circuit (24) to operate a suitable alarm (25).

15. An apparatus as claimed in Claim 14 and wherein the constituents connected to take output from said linear voltage displacement transducer (18) and operate said alarm (25) are replaced by a microprocessor programmed to perform the component functions.

16. A method of operating a device as claimed in Claim 8 wherein the indications are produced by sampling at regular intervals from the time that pressure is first applied to said part and continuing beyond the point in time at which the stable state of the tubing is reached.

17. A method as claimed in Claim 16 wherein an indication produced by sampling is compared with an immediately preceding indication produced by sampling.

18. A method as claimed in Claim 16 or 17 wherein the rate of sampling is approximately once per second.

## Patentansprüche

1. Pumpvorrichtung zur Aufnahme eines Schlauchs (1), wobei die Vorrichtung folgendes aufweist: eine Druckaufbringeinrichtung (5), um auf in dem Schlauch (1) befindliches Fluid Druck aufzubringen, eine erste und eine zweite steuerbare Ventileinrichtung (6, 7) jeweils an der Eingangs- und der Ausgangsseite der Druckaufbringeinrichtung (5), um den Fluiddurchfluß zu begrenzen, eine Detektiereinrichtung (9), um Fluiddruck in dem Schlauch zu detektieren, die an der Ausgangsseite der zweiten Ventileinrichtung (7) positioniert ist, und eine Steuereinrichtung (8), die auf die Detektiereinrichtung (9) anspricht und betätigbar ist, um die beiden Ventileinrichtungen (6, 7) zu steuern, dadurch gekennzeichnet, daß die Steuereinrichtung (8) Mittel aufweist, die betätigbar sind, um die beiden Ventileinrichtungen (6, 7) zum Öffnen zu veranlassen, wenn Erhöhungen des Fluiddrucks, die einen gegebenen Schwellenwert überschreiten, in dem Schlauch (1) detektiert werden, so daß in dem Schlauch (1) befindliches Fluid unter Überdruck zurück zu der ersten Ventileinrichtung (6) abgelassen wird.

2. Vorrichtung nach Anspruch 1, wobei die Steuereinrichtung (8) betätigbar ist, um aufgrund von Erhöhungen des Fluiddrucks in dem Schlauch, die von der Detektiereinrichtung (9) detektiert werden, die Druckaufbringeinrichtung (5) zu desaktivieren.

3. Vorrichtung nach Anspruch 1 oder 2, wobei jede Ventileinrichtung (6, 7) betätigbar ist, um den Schlauch (1) zusammenzudrücken, um einen Verschluß desselben hervorzurufen.

4. Vorrichtung nach einem der Ansprüche 1 bis 3 in Kombination mit einem Schlauch (1), der zwischen einer Fluidquelle (2) und einer Anwendungsstelle (4) des Fluids angeschlossen ist.

5. Pumpvorrichtung nach einem der vorhergehenden Ansprüche, wobei die Druckaufbringeinrichtung (5) ein erstes und ein zweites Element (26, 27) aufweist, die eine den Schlauch (1) aufnehmende Kammer definieren, wobei die Elemente (26, 27) relativ hin- und herbewegbar sind, um eine abwechselnde Verformung und Rückbildung des Schlauchs (1) bei einem Pumpvorgang zu bewirken, wobei das Volumen des Schlauchs (1) verringert wird, wenn er verformt wird, wobei die Vorrichtung Mittel aufweist, um den Schlauch (1) in seinen rückgebildeten Zustand zu drängen, in dem die normale Querschnittsgestalt des Schlauchs (1) wiederhergestellt ist, wobei in einer ersten relativen Position der Elemente (26, 27) die Kammer im wesentlichen zylindrisch ist, so daß die Elemente (26, 27) den Schlauch (1) in seinen rückgebildeten Zustand drängen, und die Elemente (26, 27) in einer Radialrichtung der Kammer und des Schlauchs (1) in eine zweite relative Position relativ bewegbar sind, in der die Kammer langgestreckt ist, um die Verformung des Schlauchs (1) zu bewirken.

6. Pumpvorrichtung nach Anspruch 5, wobei die Steuereinrichtung (8) betätigbar ist, um die Hin- und Herbewegung der Elemente (26, 27) und den Betrieb der Ventileinrichtungen zu steuern, so daß, wenn der Schlauch von den Elementen (26, 27) verformt wird, die Ventileinrichtung (6) an der Aufstromseite den Fluiddurchfluß durch den Schlauch (1) drosselt, während die Ventileinrichtung (7) an der Abstromseite einen erhöhten Flüssigkeitsdurchfluß zuläßt, und, wenn der Schlauch in seinen rückgebildeten Zustand gedrängt wird, die Ventileinrichtung (7) an der Abstromseite den Fluiddurchfluß durch den Schlauch (1) drosselt, während die Ventileinrichtung (6) an der Aufstromseite einen erhöhten Flüssigkeitsdurchfluß zuläßt.

7. Pumpvorrichtung nach einem der vorhergehenden Ansprüche, wobei die Detektiereinrichtung (9) folgendes aufweist: eine erste Einrichtung (11, 16), um den Schlauch (1) zu veranlassen, über eine Länge davon einen länglichen Querschnitt anzunehmen, um einen relativ abgeflachten Bereich einer Wand des Schlauchs (1) innerhalb dieser Länge zu definieren, eine zweite Einrichtung (18, 19), um auf einen Teil des relativ abgeflachten Bereichs Druck aufzubringen, wobei die zweite Einrichtung auf eine Bewegung dieses Teils anspricht, um Anzeigen von Änderungen des Drucks des Fluids zu liefern, und eine Einrichtung (20-25) zum Ausgleich der Bewegung des genannten Teils, die nach dem Aufbringen von Druck darauf erfolgt und nicht auf Änderungen des Drucks des Fluids zurückgeht.

8. Pumpvorrichtung nach Anspruch 7, wobei die Ausgleichseinrichtung (20-25) aufweist: Mittel (23) zum Vergleichen einer zu einem gegebenen Zeitpunkt erhaltenen Anzeige mit einer zu einem vorhergehenden Zeitpunkt erhaltenen Anzeige und Mittel (24), die auf das Vorhandensein einer Differnez zwischen beiden oberhalb eines Schwellenpegels ansprechen, um anzuzeigen, daß eine Änderung des Drucks des Fluids eingetreten ist.

9. Vorrichtung nach Anspruch 7 oder 8, wobei die erste Einrichtung (11, 16), die den Schlauch (1) veranlaßt, über eine Länge davon einen länglichen Querschnitt anzunehmen, folgendes aufweist: ein Bettelement (11), das eine unelastische, im wesentlichen flache Oberfläche hat, über die der Schlauch (1) positioniert werden kann, und ein Körperelement (14, 16), um den Schlauuch (1) nichtverschließend über eine Länge davon gegen die Oberfläche des Bettelements abzuflachen, wobei die zweite Einrichtung (18, 19) ein Druckaufbringelement (18) aufweist, das an dem Körperelement (14, 16) getragen ist, um Druck auf eine Stelle in dem relativ abgeflachten Bereich aufzubringen.

10. Vorrichtung nach Anspruch 9, wobei das Druckaufbringelement (18) einen Wegmeßumformer mit linearer Spannung aufweist, der einen federbeaufschlagten Plunger (19) hat und im Betrieb ein Ausgangsspannungssignal erzeugt, das den Weg bezeichnet, um den sich der Plunger über den Körper des Meßumformers hinaus erstreckt, wobei der Plunger (19) angeordnet ist, um in den relativ abgeflachten Bereich der Wand des Schlauchs an der genannten Stelle gedrückt zu werden.

11. Vorrichtung nach Anspruch 10, wobei der Wegmeßumformer (18) in dem Körperelement (14, 16) untergebracht ist, das angeordnet ist, um zu und von dem Bettelement (11) bewegbar zu sein, so daß der Schlauch (1) mit dem Plunger (19) in Eingriff gelangen oder davon freigegeben werden kann.

12. Vorrichtung nach Anspruch 11, wobei das Körperelement (14, 16) einen rohrförmigen Vorsprung aufweist, der sich über einen zylindrischen Kanal (15) innerhalb des Körperelements hinaus erstreckt, wobei der Kanal in eine Oberfläche des Körperelements, die dem Bettelement (11) zugewandt ist, mündet und der rohrförmige Vorsprung (16) dazu dient, den Schlauch wie angegeben abzuflachen, wenn das Körperelement (14, 16) in Richtung zu dem Bettelement (11) bewegt wird.

13. Vorrichtung nach Anspruch 11 oder 12, wobei das Körperelement (14, 16) und das Bettelement (11) gelenkig miteinander verbunden sind und Mittel vorgesehen sind, um das eine an dem anderen zu befestigen, wenn das Körperelement im Betrieb bis zu dem Bettelement hin geschlossen wird.

14. Vorrichtung nach einem der Ansprüche 10 bis 13, wobei das Ausgangssignal des Wegmeßumformers (18) mit linearer Spannung angeordnet ist, um von einer Abtastschaltung (20) abgetastet zu werden, deren Ausgangssignal über einen Analog-Digital-Wandler (21) mit dem Eingang eines einseitig gerichteten Schieberegisters (22) und parallel mit einem Eingang einer Komparatorschaltung (23) verbunden ist, deren zweites Eingangssignal vom Ausgang des Schieberegisters (22) abgeleitet ist, so daß das digitalisierte Ausgangssignal der Abtastschaltung mit dem unmittelbar vorhergehenden Ausgangssignal der Abtastschaltung, das von dem Schieberegister abgegeben wird, verglichen wird, wobei das Ausgangssignal der Komparatorschaltung (23) über eine Schwellenwertschaltung (24) gekoppelt ist, um einen geeigneten Alarm (25) zu betätigen.

15. Vorrichtung nach Anspruch 14, wobei die Bauelemente, die gekoppelt sind, um das Ausgangssignal des Wegmeßumformers (18) mit linearer Spannung aufzunehmen und den Alarm (25) zu betätigen, durch einen Mikroprozessor ersetzt sind, der zur Durchführung der Funktionen der Bauelemente programmiert ist.

16. Verfahren zum Betätigen einer Vorrichtung nach Anspruch 8, wobei die Anzeigen erzeugt werden durch Abtasten in regelmäßigen Intervallen ab dem Zeitpunkt, zu dem Druck erstmals auf den Teil aufgebracht wird, und Fortfahren über den Zeitpunkt hinaus, zu dem der stabile Zustand des Schlauchs erreicht ist.

17. Verfahren nach Anspruch 16, wobei eine durch Abtasten erzeugte Anzeige mit einer unmittelbar vorhergehenden, durch Abtasten erzeugten Anzeige verglichen wird.

18. Verfahren nach Anspruch 16 oder 17, wobei die Abtastrate ungefähr einmal pro Sekunde ist.

## Revendications

1. Agencement de pompage destiné à recevoir un tube (1) d'agencement comprenant un moyen de mise sous pression (5) pour soumettre à une pression le liquide dans le tube (1), des première et deuxième vannes contrôlables (6, 7) respectivement sur le côté d'entrée et le côté de sortie du moyen de mise sous pression (5) afin de restreindre le passage du liquide, un moyen de détection (9) pour détecter la pression du liquide dans le tube (1) et disposé sur le côté de sortie de ladite deuxième vanne (7), et un moyen de commande (8) réagissant audit moyen de détection (9) et pouvant être actionné pour commander chacune desdites vannes (6, 7), caractérisé en ce que le moyen de commande comprend un moyen (8) pouvant être actionné pour provoquer l'ouverture des deux vannes (6, 7) lorsqu'une augmentation de pression du fluide dépassant un seuil déterminé est détectée dans le tube (1), tandis que le liquide sous pression excessive dans ledit tube (1) est renvoyé vers la première vanne (6).

2. Agencement selon la revendication 1, dans lequel le moyen de commande (8) peut être actionné pour mettre hors service ledit moyen de mise sous pression (5) en réponse à une augmentation de la pression du liquide dans le tube détectée par ledit moyen de détection (9).

3. Agencement selon la revendication 1 ou 2, dans lequel chaque vanne (6, 7) peut être actionnée pour écraser ledit tube (1) de manière à produire l'occlusion de celui-ci.

4. Agencement selon l'une quelconque des revendications 1 à 3 en combinaison avec le tube (1) connecté entre une source de liquide (2) et un point (4) d'application du liquide.

5. Agencement de pompage selon l'une quelconque des revendications précédentes, dans lequel le moyen de mise sous pression (5) comprend un premier et un deuxième éléments (26, 27) définissant une chambre recevant le tube (1), les éléments (26, 27) pouvant se déplacer d'un mouvement relatif de va-et-vient de manière à provoquer une déformation et une remise en forme alternées du tube (1) au cours d'une action de pompage dans laquelle le volume du tube (1) est diminué lorsqu'il est déformé, l'appareil comprenant un moyen pour ramener le tube (1) à son état de remise en forme dans lequel est rétablie la section normale transversale du tube (1), dans lequel, dans une première position relative des éléments (26, 27), la chambre est de forme essentiellement cylindrique alors que les éléments (26, 27) poussent le tube (1) dans son état de remise en forme et que les éléments (26, 27) peuvent se déplacer d'un mouvement relatif dans une direction radiale par rapport à la chambre et au tube (1) jusqu'à une deuxième position relative, dans laquelle la chambre est allongée de manière à provoquer la déformation du tube (1).

6. Agencement de pompage selon la revendication 5, dans lequel ledit moyen de commande (8) peut être actionné pour commander le mouvement de va-et-vient desdits éléments (26, 27) et le fonctionnement desdites vannes de façon que, lorsque ledit tube est déformé par lesdits éléments (26, 27), la vanne (6) sur le côté amont restreint le passage du liquide à travers ledit tube (1) tandis que la vanne (7), sur le côté aval, permet un débit de liquide accru et, lorsque le tube est poussé pour le ramener à sa forme initiale, la vanne (7) sur le côté aval restreint le passage du liquide à travers le tube (1) tandis que la vanne (6) sur le côté amont permet un débit de liquide augmenté.

7. Agencement de pompage selon l'une quelconque des revendications précédentes, dans lequel le moyen de détection (9) comprend un premier moyen (11, 16) pour donner au tube (1) une section transversale allongée sur une certaine longueur de celui-ci, de manière à former une région relativement aplatie d'une paroi du tube (1) dans ladite longueur, un deuxième moyen (18, 19) pour appliquer une pression à une partie de la région relativement aplatie le deuxième moyen réagissant au mouvement de ladite partie en fournissant des indications des variations de pression dudit liquide, et un moyen (20, 25) pour compenser le déplacement de ladite partie qui se produit après que ladite pression y a été appliquée et dû à d'autres variations de la pression dudit liquide.

8. Agencement de pompage selon la revendication 7, dans lequel ledit moyen de compensation (25) comprend un moyen (23) pour comparer une indication obtenue à un moment donné avec une indication obtenue à un moment précédent, et un moyen (24) réagissant à l'existence entre ces indications d'une différence supérieure à un niveau de seuil pour signaler qu'un changement de la pression dans le liquide s'est produit.

9. Agencement selon la revendication 7 ou 8, et dans lequel ledit premier moyen (11, 16) pour donner audit tube (1) une section transversale allongée sur une certaine longueur de celui-ci comprend un élément de lit (11) ayant une surface non élastique essentiellement plate sur laque-le peut être placé ledit tube (1), et un élément de corps (14, 16) pour aplatir ledit tube (1) sans le fermer sur une certaine longueur de celui-ci contre ladite surface dudit élément de lit, et le deuxième moyen (18, 19) comprend un élément d'application de pression (18) supporté par l'élément de corps (14, 16) pour appliquer une pression à un point dans ladite région relativement aplatie.

10. Agencement selon la revendication 9 et dans lequel ledit élément appliquant la pression (18) comprend un transducteur de déplacement de tension linéaire ayant un plongeur (19) chargé par ressort et produisant, en fonctionnement, un signal de tension de sortie qui indique le degré d'extension dudit plongeur au-delà du corps dudit transducteur, ledit plongeur (19) étant disposé de manière à être poussé dans la région relativement aplatie de la partie dudit tube à cet endroit.

11. Agencement selon la revendication 10 et dans lequel ledit transducteur (18) est logé dans l'élément de corps (14, 16), qui est conçu de manière à se déplacer en se rapprochant et en s'écartant dudit élément de lit (11), dans lequel ledit tube (1) peut venir en contact avec ledit plongeur (19) ou se dégager de celui-ci.

12. Agencement selon la revendication 11 et dans lequel ledit élément de corps (14, 16) comprend une saillie tubulaire s'étendant au-delà d'un passage cylindrique (15) dans ledit élément de corps, ledit passage s'ouvrant dans une surface dudit élément de corps tournée vers ledit élément de lit (11) et ladite saillie tubulaire (16) servant à aplatir ledit tube, comme déjà mentionné, lorsque ledit corps (14, 16) est déplacé vers ledit élément de lit (11).

13. Agencement selon la revendication 11 ou 12, et dans lequel ledit élément de corps (14, 16) et ledit élément de lit (11) sont articulés l'un à l'autre, et dans lequel des moyens sont prévus pour les fixer l'un à l'autre lorsque l'élément de corps est rapproché dudit élément de lit en fonctionnement.

14. Agencement selon l'une quelconque des revendications 10 à 13, et dans lequel la sortie dudit transducteur de déplacement de tension linéaire (18) est conçu pour être échantillonné par un circuit d'échantillonnage (20), dont la sortie est connectée par un convertisseur analogique-numérique (21) à l'entrée d'un registre de déplacement unidirectionnel (22) et en parallèle à une entrée d'un circuit de comparaison (23), dont une deuxième entrée est dirigée de la sortie dudit registre de déplacement (22), dans lequel la sortie numérisée dudit circuit d'échantillonnage est comparée avec la sortie immédiatement précédente dudit circuit d'échantillonnage provenant dudit registre de déplacement, la sortie dudit circuit de comparaison (23) étant connectée par un circuit de seuil (24), de manière à actionner une alarme appropriée (25).

15. Agencement selon la revendication 14 et dans lequel les constituants connectés pour recevoir une sortie provenant dudit transducteur de déplacement de tension linéaire (18) et pour actionner ladite alarme (25) sont remplacés par un microprocesseur programmé pour effectuer les fonctions des constituants.

16. Procédé d'utilisation d'un dispositif selon la revendication 8, dans lequel les indications sont obtenues en échantillonnant à des intervalles réguliers, depuis le moment où la pression est appliquée pour la première fois à ladite pièce et, ensuite, continuellement jusque après le moment où est atteint l'état stable du tube.

17. Procédé selon la revendication 16, dans lequel une indication produite par échantillonnage est comparée avec une indication immédiatement précédente produite par échantillonnage.

18. Procédé selon la revendication 16 ou 17, dans lequel la vitesse d'échantillonnage est égale à environ une fois par seconde.
